# EUROPÄISCHE PATENTSCHRIFT

(11) **EP 3 818 073 B1**
(45) Veröffentlichungstag und Bekanntmachung des Hinweises auf die Patenterteilung: **03.12.2025**
(21) Anmeldenummer: 18740169.0
(22) Anmeldetag: 06.07.2018
(51) Int. Cl.: C07K 14/245, C12P 21/02, C12N 15/70

(54) **BAKTERIENSTAMM ZUR FREISETZUNG EINES REKOMBINANTEN PROTEINS IN EINEM FERMENTATIONSVERFAHREN**
BACTERIAL STRAIN FOR RELEASING A RECOMBINANT PROTEIN IN A FERMENTATION METHOD
SOUCHE BACTÉRIENNE POUR LIBÉRER UNE PROTÉINE RECOMBINANTE DANS UN PROCÉDÉ DE FERMENTATION

(43) Veröffentlichungstag der Anmeldung: 12.05.2021
(73) Patentinhaber: Wacker Chemie AG, 81671 München (DE)
(72) Erfinder: KOCH, Johanna, 81377 München (DE); BRUNNER, Markus, 86415 Mering (DE)
(74) Vertreter: Belz, Ferdinand
(86) Internationale Anmeldenummer: PCT/EP2018/068418
(87) Internationale Veröffentlichungsnummer: WO 2020/007493

(56) Entgegenhaltungen:
- EP-A1- 1 905 839
- EP-A1- 2 204 441
- ALEXANDRA GENNARIS ET AL: "Repairing oxidized proteins in the bacterial envelope using respiratory chain electrons", NATURE, vol. 528, no. 7582, 7 December 2015 (2015-12-07), London, pages 409 - 412, XP055351255, ISSN: 0028-0836, DOI: 10.1038/nature15764
- ALEXANDRA GENNARIS ET AL: "Repairing oxidized proteins in the bacterial envelope using respiratory chain electrons", NATURE, vol. 528, no. 7582, 7 December 2015 (2015-12-07), London, pages 409 - 412, XP055562384, ISSN: 0028-0836, DOI: 10.1038/nature15764
- CLAVEL THIERRY ET AL: "TolB protein of Escherichia coli K-12 interacts with the outer membrane peptidoglycan-associated proteins Pal, Lpp and OmpA", MOLECULAR MICROBIOLOGY, vol. 29, no. 1, July 1998 (1998-07-01), pages 359 - 367, XP055562368, ISSN: 0950-382X
- ZHAO-YUAN CHEN ET AL: "Construction of leaky strains and extracellular production of exogenous proteins in recombinant E scherichia coli : Extracellular production of exogenous proteins", MICROBIAL BIOTECHNOLOGY, vol. 7, no. 4, 30 April 2014 (2014-04-30), GB, pages 360 - 370, XP055561732, ISSN: 1751-7915, DOI: 10.1111/1751-7915.12127

## Beschreibung

Hierin beschrieben ist ein Bakterienstamm enthaltend einen offenen Leserahmen codierend für ein rekombinantes Protein unter Kontrolle eines funktionellen Promotors dadurch gekennzeichnet, dass der Bakterienstamm einen offenen Leserahmen codierend für ein mutiertes Peptidoglykan-assoziiertes Lipoprotein (Pal-Protein) unter Kontrolle eines funktionellen Promotors enthält, wobei das Pal-Protein derart mutiert ist, dass es keinen Membrananker für die äußere Zellmembran des Bakteriums besitzt. Hierin auch beschrieben ist ein Plasmid codierend ein rekombinantes Protein und das mutierte Pal-Protein sowie ein Verfahren zur fermentativen Produktion von rekombinanten Proteinen unter Verwendung des Bakterienstammes.

Rekombinante Proteine können in Bakterien, wegen ihrer kurzen Generationszeit und der einfachen Handhabung, im Vergleich zu Säugerzellkulturen kostengünstig hergestellt werden. Aufgrund seiner sehr gut untersuchten Genetik und Physiologie ist das gramnegative Enterobakterium *Escherichia coli* gegenwärtig der am häufigsten verwendete Organismus zur Produktion rekombinanter Proteine. Besonders attraktiv sind Produktionsverfahren für rekombinante Proteine in *E. coli,* bei denen das Zielprotein in hoher Ausbeute und in der korrekten Faltung direkt ins Fermentationsmedium freigesetzt wird, da hier aufwändige Zellaufschluss- und Proteinfaltungsprozesse vermieden werden. In der Literatur sind eine Reihe von *E. coli*-Stämmen und Prozesse mit *E. coli*-Stämmen offenbart, durch die eine Freisetzung von rekombinanten Proteinen ins Kulturmedium erreicht wird.
i) Zunächst besteht die Möglichkeit der Verwendung von sogenannten "leaky"-Stämmen. Darunter sind Mutanten von *E. coli* zu verstehen, die einen Defekt in der äußeren Membran aufweisen und deshalb periplasmatische Proteine teilweise in das Kulturmedium entlassen. Es handelt sich dabei um einen unspezifischen Mechanismus. Ein Beispiel für solche "leaky"-Mutanten sind Stämme mit Defekten im Tol-Pal-Komplex, z.B. tol- und pal-Deletions-Mutanten. Es ist bekannt, dass tol- und pal-Deletions-Mutanten zelleigene periplasmatische Proteine, wie z.B. die alkalische Phosphatase PhoA oder die RNase I, in das Fermentationsmedium entlassen. Solche Stämme sind extrem empfindlich gegenüber EDTA, verschiedenen Detergenzien und Antibiotika und weisen Wachstumsdefekte auf (Lazzaroni und Portalier 1981, J. Bact. 145, S. 1351-1358; Lazzaroni and Portalier 1992, Mol. Microbiol. 6, S. 735-742; Chen et al. 2014, Microb. Biotechnol. 7, S. 360-370; Bernstein et al. 1972, J. Bacteriol. 112, S. 74-83). Daher sind sie zur Kultivierung unter Hochzelldichte-Fermentations-Bedingungen nur eingeschränkt geeignet.
ii) Ein weiterer Ansatz, um die Freisetzung von rekombinanten Proteinen zu erreichen, ist die Expression eines Proteins, das die Zellhülle von *E. coli* permeabilisiert und so die Freisetzung von Proteinen ins Kulturmedium begünstigt. Gut beschrieben ist die Expression von Bacteriocin Release Proteinen (BRPs), kleinen Lipoproteinen, die zu einem Abbau der äußeren Membran und zur Lyse der Zellen führen. Durch Überexpression des ColE1-BRP (Kil-Protein) oder des Cloacin DF13 BRP konnten Interleukin-2, β-Glukanase, Alkalische Phosphatase, oder β-Laktamase in das Kulturmedium entlassen werden (Beshay et al. 2007, Biotechnol. Lett. 29, S. 1893-1901; Robbens et al. 1995, Protein Expr. Purif. 6, S. 481-486; Sommer et al. 2010, J. Biotechnol. 145, S. 350-358).
iii) Ein anderer Ansatz zur Destabilisierung der Zellhülle ist in Wan und Baneyx (1998, Protein Expr. Purif. 14, S. 13-22) beschrieben. Hier wurde die Membranintegrität gestört, indem die lösliche, C-terminale Domäne des TolA-Proteins (TolAIII) überexprimiert wurde. Dies führte zu einem ähnlichen Phänotyp wie in tolA-Deletions-Mutanten mit verstärkter Freisetzung der Proteine RNaseI und Alkalische Phosphatase aus dem Periplasma in das Medium, sowie Sensitivität gegenüber Desoxycholat (Levengood-Freyermuth et al. 1993, J. Bacteriol. 175, S. 222-228). Durch Überexpression des TolAIII-Proteins wurde die Freisetzung eines OmpA-TEM-β-Laktamase-Proteins deutlich gesteigert. Die Expression von β-Laktamase ging im Vergleich zur Kontrolle jedoch um Faktor 1,5-2 zurück. Weiterhin war die Vitalität der Zellen durch Expression von TolAIII, genau wie in tolA-Mutanten, stark beeinträchtigt. Die Zahl der Kolonie-formenden Einheiten (CFU, colony forming units), als Maß der Vitalität der Kultur, ging bereits 3 h nach Beginn der TolAIII-Expression im Schüttelkolben um den Faktor 1000 zurück. Außerdem waren die Zellen sensitiv gegenüber geringen Konzentrationen an SDS (0,02 %), was auf deutliche Membrandefekte hindeutet.

Solche Zellen sind für eine Kultivierung bei hohen Zelldichten, über einen längeren Zeitraum, der zur Produktion komplexer, eukaryontischer Proteine notwendig ist, nicht geeignet. Ein weiterer Nachteil der Coexpression von TolAIII ist, dass TolAIII selbst in großen Mengen im Kulturüberstand zu finden ist und so das ausgeschleuste Zielprotein verunreinigt.

Aufgabe der vorliegenden Erfindung ist es, einen Bakterienstamm bereitzustellen, der ein rekombinantes Protein in erhöhter Ausbeute in das Kulturmedium freisetzt, ohne dass es zu einer starken Zelllyse kommt.

Diese Aufgabe wird gelöst durch einen Bakterienstamm enthaltend einen offenen Leserahmen codierend für ein rekombinantes Protein unter Kontrolle eines funktionellen Promotors, dadurch gekennzeichnet, dass er einen offenen Leserahmen codierend für ein mutiertes Peptidoglykan-assoziiertes Lipoprotein (Pal-Protein) unter Kontrolle eines funktionellen Promotors enthält, wobei das mutierte Pal-Protein derart mutiert ist, dass es keinen Membrananker für die äußere Zellmembran des Bakteriums besitzt.

Die Erfindung ist in den beigefügten Ansprüchen definiert.

Die Verwendung des oben definierten Bakterienstammes in einem Fermentationsverfahren hat den Vorteil, dass zusammen mit dem rekombinanten Protein ein zusätzliches Protein, nämlich eine mutierte Form des Pal-Proteins exprimiert wird. Durch die Expression des mutierten Pal-Proteins kommt es zu einer eingeschränkten Permeabilisierung der äußeren Zellmembran des Bakteriums, so dass rekombinante Proteine aus der Zelle freigesetzt werden können, ohne dass die Zelle abstirbt. Durch die verbesserte Freisetzung lassen sich die rekombinanten Proteine in erhöhter Ausbeute isolieren. Die Offenbarung stellt einen entsprechenden Bakterienstamm, ein Verfahren zur fermentativen Produktion von rekombinanten Proteinen unter Verwendung dieses Bakterienstammes sowie ein entsprechendes Plasmid zur Verfügung. Auf diese Weise können gegenüber dem Stand der Technik erhöhte Produktausbeuten im Kulturüberstand erzielt werden, ohne dass es zu einem beträchtlichen Absterben der Bakterienzellen kommt.

Der Bakterienstamm ist bevorzugt dadurch gekennzeichnet, dass es sich bei dem Bakterienstamm um gramnegative Bakterien, besonders bevorzugt um einen Bakterienstamm der Gattung Enterobacteriaceae, insbesondere bevorzugt um einen Stamm der Art *Escherichia* coli handelt.

Beim Peptidoglykan-assoziierten Lipoprotein (Pal, Pal-Protein) handelt es sich um ein bakterielles, periplasmatisches Protein, das mit seinem lipidierten N-Terminus in der äußeren Membran der Bakterienzelle verankert ist und mit dem C-Terminus mit der Peptidoglykanschicht interagiert. Außerdem ist Pal ein Bestandteil des Pal-Tol-Systems (Godlewska et al. 2009, FEMS Microbiol. Lett. 298, S. 1-11) und interagiert mit diversen weiteren Proteinen des Periplasmas, etwa lpp und ompA (Cascales et al., 2002, J. Bacteriol. 184, S. 754-759; Godlewska s.o.). Das Pal-Protein wird in der technischen Anwendung zum Beispiel zur Verankerung von Antikörper-Fragmenten, in Form eines Fusionsproteins aus Pal-Protein und Antikörper-Fragment, auf der Zelloberfläche von Bakterien verwendet (Fuchs et al. 1991, Biotechnology (N Y) 9, S. 1369-1372; Dhillon et al. 1999, Lett. Appl. Microbiol. 28, S. 350-354).

Als Wildtyp-Pal-Gen wird die Form des Pal-Gens bezeichnet, die natürlicherweise durch die Evolution entstanden und im Wildtyp-Bakteriengenom vorhanden ist. Vom Wildtyp-Pal-Gen wird das Wildtyp-Pal-Vorläufer-Protein exprimiert, das ein Signalpeptid und die Aminosäuresequenz des Wildtyp-Pal-Proteins umfasst (SEQ ID Nr. 4). Nach Abspalten der Signalsequenz von 21 Aminosäuren umfasst das reife Wildtyp-Pal-Protein aus *E. coli* (Stamm K-12) eine Sequenz von 152 Aminosäuren (s. Genbankeintrag X05123, Uniprot P0A912) und trägt an Aminosäureposition 1 die in bakteriellen Lipoproteinen hoch-konservierte Aminosäure Cystein, die mit dem Membrananker acyliert ist (s. z.B. Zückert et al. 2014, Biochimica et Biophysica Acta 1843, S. 1509-1516 oder Konovalova und Silhavy 2015, Phil. Trans. R. Soc. B 370: 20150030). Dagegen besitzt das mutierte Pal-Protein keinen Membrananker für die äußere Zellmembran des Bakteriums.

Als Pal-Vorläufer-Protein (Vorläufer-Form des Pal-Proteins) wird das Pal-Protein bezeichnet, das die Aminosäuresequenz des Pal-Proteins und die Signalsequenz umfasst und keine posttranslationalen Modifikationen trägt.

Als reifes Wildtyp-Pal-Protein wird das Pal-Protein bezeichnet, das die Aminosäuresequenz des Wildtyp-Pal-Proteins umfasst und keine Signalsequenz mehr trägt, da diese abgespaltet worden ist, und bei dem eventuelle posttranslationale Modifikationen vorhanden sind. Eine solche posttranslationale Modifikation ist beispielsweise die Acylierung des Pal-Proteins mit dem Membrananker.

Als offener Leserahmen (*open reading frame,* ORF) wird derjenige Bereich der DNS bzw. RNS bezeichnet, der zwischen einem Startcodon und einem Stoppcodon liegt und für die Aminosäuresequenz eines Proteins codiert. Der ORF wird auch als codierende Region bezeichnet.

ORFs werden von nicht-codierenden Bereichen umgeben. Als Gen wird der DNS-Abschnitt bezeichnet, der alle Grundinformationen zur Herstellung einer biologisch aktiven RNS enthält. Ein Gen enthält den DNS-Abschnitt, von dem durch Transkription eine einzelsträngige RNS-Kopie hergestellt wird und die Expressionssignale, die an der Regulation dieses Kopiervorgangs beteiligt sind. Zu den Expressionssignalen zählen z.B. mindestens ein Promotor, ein Transkriptions-, ein Translationsstart und eine Ribosomenbindestelle. Des Weiteren sind als Expressionssignale ein Terminator und ein oder mehrere Operatoren möglich.

Für einen funktionellen Promotor gilt, dass der unter der Regulation dieses Promotors stehende ORF in eine RNS transkribiert wird.

Als monocistronisch wird eine Boten-RNS (mRNA) bezeichnet, die nur einen offenen Leserahmen enthält.

Ein Operon ist eine Funktionseinheit der DNS, die mehrere ORFs, einen Promotor und gegebenenfalls weitere Expressionssignale umfasst.

Der Bakterienstamm kann (1) einen ORF codierend für ein rekombinantes Protein, (2) mehrere ORFs codierend für dasselbe rekombinante Protein oder (3) mehrere ORFs codierend für verschiedene rekombinante Proteine enthalten. Während die 2. Möglichkeit z.B. zur Erhöhung der Expression und Ausbeute des rekombinanten Proteins verwendet wird, findet die 3. Möglichkeit besonders bei der Expression von Proteinen, die aus mehreren Polypeptiden (Untereinheiten) aufgebaut sind, Anwendung. Ein Beispiel ist die Expression von Antikörperfragmenten, die dann nach dem Transport aus dem Cytoplasma assembliert werden. Jeder dieser ORFs kann in einem separaten Gen liegen, es können auch mehrere ORFs in einem Operon organisiert sein, d.h. von gemeinsamen Expressionssignalen reguliert werden.

Jedes Operon kann zudem einen ORF codierend das mutierte Pal-Protein enthalten. Auch wenn ein oder mehrere ORFs codierend ein oder mehrere rekombinante Proteine und der ORF codierend das mutierte Pal-Protein in einem Operon liegen, wird das mutierte Pal-Protein immer als separates Protein und nicht als Fusionsprotein exprimiert, da sich der ORF codierend das mutierte Pal-Protein definitionsgemäß durch ein eigenes Translationsstart- und Stoppcodon auszeichnet.

Im Fall, dass das rekombinante Protein aus mehreren Polypeptidketten (Untereinheiten) aufgebaut ist, ist es bevorzugt, dass die ORFs der einzelnen Peptidketten (Untereinheiten) in einem Operon organisiert sind.

Es ist bevorzugt, dass der ORF codierend das mutierte Pal-Protein als monocistronische Boten-RNS transkribiert wird. D. h., dass das mutierte Pal-Protein von einem separaten, eigenen Gen exprimiert wird. Dieses Gen wird als mutiertes Pal-Gen bezeichnet.

Die ORFs codierend das rekombinante Protein und das mutierte Pal-Protein können chromosomal oder von einem Plasmid exprimiert werden. Wenn es sich um getrennte Gene handelt, können diese auch von getrennten, in Bezug auf Replikationsursprung und Selektionsmarker miteinander kompatiblen, Plasmiden exprimiert werden. Bevorzugt sind die ORFs Plasmid-codiert.

Der in dieser Erfindung eingesetzte ORF codierend das mutierte Pal-Protein enthält Veränderungen in der Aminosäuresequenz wie Substitutionen, Deletionen und/oder Insertionen im Vergleich zum Wildtyp-Pal-Protein.

Gemäß der vorliegenden Erfindung handelt es sich bei der Sequenz des ORFs codierend das mutierte Pal-Protein bevorzugt um eine DNS-Sequenz, die zur Expression eines mutierten Pal-Proteins führt, die aus dem gleichen Organismus, der auch zur Produktion der rekombinanten Proteine verwendet wird, stammt.

Der Ursprung der DNS-Sequenz des erfindungsgemäßen ORFs codierend das mutierte Pal-Protein ist nicht beschränkt auf den zur Produktion des rekombinanten Proteins verwendeten Bakterienstamm, solange das entsprechende Pal-Protein in der nicht-mutierten Form in dem zur Produktion des rekombinanten Proteins verwendeten Bakterienstamm funktionell ist. Funktionell bedeutet in diesem Zusammenhang, dass alle biologischen Funktionen des Pal-Proteins des zur Produktion des rekombinanten Proteins verwendeten Bakterienstamms vom oben genannten Pal-Protein übernommen werden können. Insbesondere bedeutet dies, dass die phänotypischen Ausprägungen eines Bakterienstammes mit einer chromosomalen Deletion des Pal-Gens von einer zusätzlich eingebrachten Kopie des oben genannten Pal-Gens komplementiert werden können.

Der im Rahmen dieser Erfindung in der Einzahl verwendete Begriff "ein/der ORF codierend für ein rekombinantes Protein" und "ein/das rekombinante/s Protein" kann auch mehrere ORFs und/oder mehrere verschiedene rekombinante Proteine bedeuten. Bevorzugt handelt es sich um 1 bis 3 verschiedene rekombinante Proteine, besonders bevorzugt um 1 rekombinantes Protein oder 2 verschiedene rekombinante Proteine. Die Klonierung und Expression von rekombinanten Proteinen in Bakterien erfolgt wie im Stand der Technik beschrieben. Das rekombinante Protein besitzt beispielsweise eine Signalsequenz für den Transport in das Periplasma.

Beim rekombinanten Protein handelt es sich um ein Protein, das vom Wildtyp-Bakteriengenom natürlicherweise entweder gar nicht oder in anderer Menge exprimiert wird. Das Bakterium dient der Herstellung des rekombinanten Proteins, wobei dieses erfindungsgemäß ins Kulturmedium freigesetzt wird.

Um die Freisetzung des rekombinanten Proteins in das Kulturmedium zu erzielen, ist es notwendig, dass sowohl das rekombinante Protein, als auch das mutierte Pal-Protein nach der Proteinbiosynthese im Cytosol in das Periplasma transportiert werden. Für den Transport in das Periplasma ist es notwendig, das 5'-Ende der codierenden DNS-Sequenz des zu produzierenden Proteins *in* frame mit dem 3'-Ende einer Signalsequenz für den Protein-Export zu verknüpfen. Dazu sind prinzipiell alle Signalsequenzen geeignet, die in dem verwendeten Bakterienstamm eine Translokation des Zielproteins mit Hilfe des Sec-, oder Tat-Apparats ermöglichen. Verschiedene Signalsequenzen sind im Stand der Technik beschrieben, so z.B. die Signalsequenzen der folgenden Gene: phoA, ompA, pelB, ompF, ompT, lamB, malE, Staphylococcal protein A, StII und andere (Choi und Lee 2004, Appl. Microbiol. Biotechnol. 64, S. 625-635). Erfindungsgemäß bevorzugt für rekombinante Proteine ist die Signalsequenz des phoA- oder des ompA-Gens von *E. coli* oder die Signalsequenz für eine Cyclodextrin-Glycosyltransferase (α-CGTase) aus *Klebsiella pneumoniae* M5a1 bzw. davon abgeleitete Signalsequenzen, die in US 2008/0076157 als SEQ ID Nr. 1 und 3 offenbart sind. Bevorzugt trägt das mutierte Pal-Protein in seiner Vorläufer-Form die native Signalsequenz des Pal-Proteins, wie in Chen und Henning (1987, Eur. J. Biochem. 163, S. 73-77) beschrieben. Bevorzugt tragen das rekombinante Protein und das mutierte Pal-Protein in ihrer Vorläufer-Form eine unterschiedliche Signalsequenz, wobei beide Signalsequenzen den Proteinexport bewirken.

Die Expression der ORFs codierend das mutierte Pal-Protein und das rekombinante Protein können von einem oder verschiedenen Promotoren kontrolliert werden. Es ist bevorzugt, dass die ORFs für das mutierte Pal-Protein und für das rekombinante Protein unter Kontrolle von verschiedenen Promotoren stehen.

Der DNS-Abschnitt codierend das mutierte Pal-Protein kann zunächst mittels PCR unter Verwendung von Oligonukleotiden als Primer und einer DNS-Matrize, codierend das Pal-Protein, beispielsweise genomische DNA isoliert aus *E. coli,* amplifiziert und anschließend mit gängigen molekularbiologischen Techniken jeweils mit dem DNS-Molekül, das die Sequenz eines Signalpeptids umfasst und das auf analoge Weise erzeugt wurde, derart verknüpft werden, dass eine in frame-Fusion entsteht. Alternativ kann auch das gesamte DNS-Molekül mittels Gensynthese hergestellt werden. Dieses DNS-Molekül bestehend aus der jeweiligen Signalsequenz und der codierenden Sequenz des mutierten Pal-Proteins kann dann entweder in einen Vektor, z.B. ein Plasmid, eingebracht werden oder direkt nach bekannten Methoden in das Chromosom des Bakterienstamms integriert werden. Bevorzugt wird das DNS-Molekül in ein Plasmid eingebracht, wie etwa einem Abkömmling von bekannten Expressionsvektoren, wie pJF118EH, pKK223-3, pUC18, pBR322, pACYC184, pASK-IBA3 oder pET. Plasmide werden unter Anwendung von dem Fachmann bekannten Methoden in die Bakterienzellen eingebracht (Transformation).

Die eingesetzten Plasmide können Selektionsmarker tragen. Als Selektionsmarker geeignet sind Gene, die für eine Resistenz gegenüber Antibiotika wie z.B. Ampicillin, Tetracyclin, Chloramphenicol, Kanamycin oder andere codieren. Bevorzugt enthält das Plasmid ein Gen, dessen Expression Tetracyclin-Resistenz vermittelt. Weiterhin sind Auxotrophie-Marker als Selektionsmarker geeignet, die für ein essentielles Gen codieren, das im jeweiligen Bakterienstamm, der das Plasmid enthält, deletiert ist. Werden zwei Plasmide in die Zellen transformiert, wird bevorzugt mit zwei verschiedenen Antibiotikaresistenzen, oder zwei verschiedenen Auxotrophie-Markern auf das Vorhandensein beider Plasmide selektiert.

Als Promotoren eignen sich alle dem Fachmann bekannten Promotoren, wie konstitutive Promotoren, wie z.B. der GAPDH-Promotor, oder induzierbare Promotoren wie z.B. der lac-, tac-, trc-, lambda PL, ara-, cumate- oder tet-Promotor oder davon abgeleitete Sequenzen. Die ORFs codierend das rekombinante Protein und der ORF codierend das mutierte Pal-Protein können als Operon von einem Promotor kontrolliert werden oder unter Kontrolle von verschiedenen Promotoren stehen. Bevorzugt werden die ORFs codierend das rekombinante Protein und der ORF codierend das mutierte Pal-Protein durch verschiedene induzierbare Promotoren kontrolliert. Besonders bevorzugt wird das rekombinante Protein unter Kontrolle des tac-Promotors und das mutierte Pal-Protein unter Kontrolle des ara (Arabinose)-Promotors exprimiert.

Die Aminosäuresequenz des mutierten Pal-Proteins lässt sich in bekannter Art und Weise herstellen, indem beispielsweise der konservierte Cystein-Rest entfernt wird, oder andere Mutationen eingeführt werden, die die Modifikation mit einem Membrananker verhindern. Dem Fachmann sind aus der Literatur Methoden bekannt, um zu überprüfen, ob eine eingeführte Mutation dazu führt, dass das mutierte Pal-Protein nach Expression in einem Bakterienstamm keinen Membrananker mehr enthält (Hayashi und Wu 1990, J. Bioenerg. Biomembr. 22, S. 451-471; Giam et al. 1984, Eur. J. Biochem. 141, S. 331-337; Lazzaroni and Portalier 1992, s.o.; Mizuno 1979, J. Biochem. 86, S. 991-1000).

In einer bevorzugten Ausführungsform ist der Bakterienstamm dadurch gekennzeichnet, dass der ORF codierend das mutierte Pal-Protein derart mutiert ist, dass er für ein mutiertes Pal-Protein codiert, das an einer oder mehreren der Aminosäurepositionen 1 bis 6, bevorzugt 1 bis 4 und besonders bevorzugt 1 bis 2 mutiert ist. Die Aminosäureposition bezieht sich dabei auf die Aminosäuren, die auf die Signalsequenz folgen, d.h. Aminosäureposition 1 ist die erste Aminosäure nach der Signalsequenz.

Bevorzugt handelt es sich bei der Mutation um eine Deletion (Fehlen von Aminosäuren) oder Substitution (Austausch von Aminosäuren), insbesondere bevorzugt um eine Deletion.

Besonders bevorzugt ist der Bakterienstamm dadurch gekennzeichnet, dass der ORF codierend das mutierte Pal-Protein derart mutiert ist, dass er für ein mutiertes Pal-Protein codiert, bei dem der N-terminale Cysteinrest substituiert ist.

In einer darüber hinaus bevorzugten Ausführungsform ist der Bakterienstamm dadurch gekennzeichnet, dass der ORF codierend das mutierte Pal-Protein derart mutiert ist, dass er für ein mutiertes Pal-Protein codiert, bei dem der N-terminale Cysteinrest fehlt. Bevorzugt wird die Aminosäure an Position 1, im speziellen bevorzugt der Cysteinrest, deletiert.

Als N-Terminus oder Amino-Terminus wird das Ende des Pal-Proteins bezeichnet, das eine freie Aminogruppe (NH₂) besitzt. Der N-terminale Cysteinrest ist der Cysteinrest an Aminosäureposition 1 nach der Signalsequenz.

Insbesondere bevorzugt ist die Aminosäuresequenz des mutierten Pal-Proteins die in SEQ ID Nr. 7 (Pal22A) angegebene Sequenz, in der das N-terminale Cystein gegen die Aminosäure Alanin ausgetauscht wurde, sodass auf die ersten 21 Aminosäuren der Signalsequenz ein Alanin folgt.

In einer bevorzugten Ausführungsform ist der Bakterienstamm, dadurch gekennzeichnet, dass der ORF codierend das mutierte Pal-Protein derart mutiert ist, dass er für ein mutiertes Pal-Protein codiert, bei dem die Aminosäuren 1 bis 6, bevorzugt 1 bis 4 und besonders bevorzugt 1 bis 2 fehlen. Insbesondere bevorzugt ist die-Aminosäure Sequenz des mutierten Pal-Proteins die in SEQ ID Nr. 5 (PalΔ₂₂₋₂₇) angegebene Sequenz, in der auf die ersten 21 Aminosäuren der Signalsequenz ein Alanin folgt. Die Sequenz nach dem Signalpeptid des mutierten Pal-Proteins ist identisch zu den Aminosäuren 7-152 des reifen Wildtyp-Pal-Proteins aus *E. coli* (Aminosäure-Sequenz des Wildtyp-Proteins s. SEQ ID Nr. 4).

Bevorzugt handelt es sich beim rekombinanten Protein um ein heterologes Protein. Unter heterologen Proteinen sind Proteine zu verstehen, die nicht zum Proteom, d.h. der gesamten natürlichen Protein-Ausstattung des Bakterienstamms, bevorzugt eines *E. coli* K12-Stamms, gehören. Alle natürlicherweise in dem verwendeten Bakterienstamm, z.B. im *E. coli* K12-Stamm, vorkommenden Proteine lassen sich aus den bekannten Genomsequenzen ableiten (z.B. aus dem Genbank-Eintrag unter der Accession No. NC_000913 für *E. coli* K12).

Als heterologe Proteine sind eukaryontische Proteine, die eine oder mehrere Disulfid-Brücken enthalten, besonders bevorzugt. Insbesondere bevorzugt sind eukaryontische Proteine, die in ihrer funktionellen Form als Di- oder Multimere vorliegen.

Zu den wichtigsten heterologen Proteinklassen zählen Antikörper und deren Fragmente, Cytokine, Wachstumsfaktoren, Proteinkinasen, Proteinhormone, Lipokaline, Antikaline, Enzyme, Bindeproteine und molekulare Scaffolds und davon abgeleitete Proteine. Beispiele für diese Proteinklassen sind u.a. heavy-chain Antikörper und deren Fragmente (z.B. Nano-bodies), einzelkettige Antikörper, Interferone, Interleukine, Interleukinrezeptoren, Interleukinrezeptor Antagonisten, G-CSF, GM-CSF, M-CSF, Leukämieinhibitoren, Stammzellenwachstumsfaktoren, Tumornekrosefaktoren, Wachstumshormone, insulinartige Wachstumsfaktoren, Fibroblastenwachstumsfaktoren, von Blutplättchen abstammende Wachstumsfaktoren, transformierende Wachstumsfaktoren, Hepatocyten-Wachstumsfaktoren, knochenmorphogenetische Faktoren, Nervenwachstumsfaktoren, vom Gehirn abstammende neurotrophe Faktoren (BDNF), von Gliazelllinien abstammende neurotrophe Faktoren, Angiogenese-Inhibitoren, Gewebeplasminogen-Aktivatoren, Blutgerinnungs-faktoren, Trypsin-Inhibitoren, Elastase-Inhibitoren, Komplementbestandteile, hypoxie-induzierte Stressproteine, Proto-Oncogen-Produkte, Transkriptionsfaktoren, viruskonstitutive Proteine, Proinsulin, Prourokinase, Erythropoietin, Thrombopoietin, Neurotrophin, Protein C, Glucocerebrosidase, Superoxid-Dismutase, Renin, Lysozym, P450, Prochymosin, Lipocortin, Reptin, Serumalbumin, Streptokinase, Tenecteplase, CNTF und Cyclodextrin-Glycosyl-Transferasen.

Beispiele für von molekularen Scaffolds abgeleitete Proteine sind u.a. Evibodies (abgeleitet von CTLA-4), Affibodies (Protein A von *S. aureus*)*,* Avimere (von humaner A-Domäne Familie), Transbodies (von Transferrin), DARPins (vom Ankyrin Repeat Protein), Adnectin (von Fibronectin III), Peptid Aptamere (von Thioredoxin), Microbodies (von Microprotein), Affiline (von Ubiquitin), α-Crystallin, Charybdotoxin, Tetranectin, PDZ Domäne des RAS-bindenden Proteins AF-6, Kunitz-Typ Domäne von Proteininhibitoren.

Bevorzugt ist der Bakterienstamm dadurch gekennzeichnet, dass dieser zusätzlich das Wildtyp-Pal-Gen codierend für das Peptidoglykan-assoziierte Lipoprotein (Pal) enthält. Besonders bevorzugt steht das Wildtyp-Pal-Gen unter Kontrolle eines funktionellen Promotors.

Ein weiterer Gegenstand der Offenbarung ist ein Plasmid enthal-tend einen offenen Leserahmen codierend für ein rekombinantes Protein unter Kontrolle eines funktionellen Promotors, dadurch gekennzeichnet, dass es einen offenen Leserahmen codierend für ein Signalpeptid und ein mutiertes Pal-Protein unter Kontrolle eines funktionellen Promotors enthält, wobei das Pal-Protein derart mutiert ist, dass es keinen Membrananker für die äußere Zellmembran des Bakteriums besitzt.

Die für den Bakterienstamm genannten bevorzugten und besonders bevorzugten Merkmale gelten auch für die im Plasmid genannten Merkmale, wobei auch im Plasmid die für den Bakterienstamm genannten bevorzugten und besonders bevorzugten Ausführungsformen jeweils bevorzugt bzw. besonders bevorzugt sind.

Ebenso gilt für den ORF codierend ein Signalpeptid und ein mutiertes Pal-Protein und den ORF codierend das rekombinante Protein die genannten Definitionen und bevorzugte Ausführungsformen.

Es können beispielsweise ein oder mehrere ORFs codierend ein oder mehrere rekombinante Proteine in einem Operon liegen. Bevorzugt liegt der ORF codierend das mutierte Pal-Protein in einem separaten Gen. Das bedeutet, dass das mutierte Pal-Protein und rekombinante Proteine unabhängig voneinander exprimiert werden können. Im besonders bevorzugten Fall, dass der oder die Promotoren des oder der rekombinanten Proteine unterschiedlich zu dem Promotor des mutierten Pal-Proteins und unterschiedlich induzierbar sind, hat das Plasmid den besonderen Vorteil, dass bei Transformation von Bakterien mit diesem Plasmid für die Expression der Proteine unabhängig voneinander der optimale Zeitpunkt gewählt werden kann.

Das Einbringen des Plasmids in den Bakterienstamm unter Anwendung von dem Fachmann bekannten Methoden und die Expression des rekombinanten Proteins und des mutierten Pal-Proteins von diesem Plasmid hat den Vorteil, dass die Freisetzung der rekombinanten Proteine aus den Zellen des Bakterienstamms verbessert wird und diese in erhöhter Ausbeute isoliert werden können.

Im Vergleich zur chromosomalen Integration der ORFs codierend das rekombinante Protein und das mutierte Pal-Protein liegt der Vorteil der Verwendung von Plasmiden darin, dass die Plasmidtragenden Zellen des Bakterienstammseinen Selektionsvorteil haben und durch Standardmethoden selektiert werden können.

Da jedes Plasmid mindestens einen Replikationsursprung (ORI) enthält, besteht ein weiterer Vorteil darin, dass Plasmide autonom replizieren. Darüber hinaus ist es von besonderem Vorteil, dass Plasmide in den Zellen des Bakterienstamms in hoher Kopienzahl vorliegen können und vererbt werden. Gegenstand der Erfindung ist ein Verfahren zur fermentativen Produktion von rekombinanten Proteinen, das dadurch gekennzeichnet ist, dass ein Bakterienstamm wie hierin beschrieben in einem Fermentationsmedium kultiviert, das Fermentationsmedium nach der Fermentation von den Zellen abgetrennt und rekombinante Proteine aus dem Fermentationsmedium isoliert werden.

Die Kultivierung der durch chromosomale Integration oder mit einem oder zwei Expressionsplasmiden transformierten Zellen des Bakterienstamms erfolgt nach üblichen, dem Fachmann bekannten Verfahren im Schüttelkolben, oder in einem Bioreaktor (Fermenter).

Als Fermentationsmedien (Nährmedien, Kulturmedien) kommen im Prinzip alle gängigen, dem Fachmann bekannten Medien für die Kultivierung von Bakterien in Frage. Dabei können Komplexmedien oder Minimalsalzmedien, denen ein bestimmter Anteil von Komplex-Komponenten, wie z.B. Pepton, Trypton, Hefeextrakt, Melasse oder Corn Steep liquor zugesetzt wird, benutzt werden. Des Weiteren können dem Medium weitere Komponenten zugegeben werden wie Vitamine, Salze, Aminosäuren und Spurenelemente, durch die das Zellwachstum verbessert wird.

Die Fermentation findet vorzugsweise in einem üblichen Bioreaktor, beispielsweise einem Rührkessel, einem Blasensäulen-Fermenter oder einem Airlift-Fermenter statt. Besonders bevorzugt ist ein Rührkessel-Fermenter.

Bei der Fermentation werden die Zellen des Protein-Produktionsstammes in einem Nährmedium kultiviert, wobei verschiedene Parameter, wie z.B. die Nährstoff-Zufuhr, der Sauerstoff-Partialdruck, der pH-Wert und die Temperatur der Kultur laufend kontrolliert und genau gesteuert werden. Der Zeitraum der Kultivierung beträgt vorzugsweise 24 - 65 h.

Als primäre Kohlenstoffquelle für die Fermentation können prinzipiell alle von den Zellen verwertbaren Zucker, Zuckeralkohole oder organische Säuren bzw. deren Salze verwendet werden. Dabei werden bevorzugt Glukose, Laktose, Arabinose oder Glycerin eingesetzt. Besonders bevorzugt sind Glukose und Arabinose. Auch ist eine kombinierte Fütterung mehrerer verschiedener Kohlenstoffquellen möglich. Die Kohlenstoffquelle kann dabei zu Beginn der Fermentation vollständig im Fermentationsmedium vorgelegt werden oder es wird nichts oder nur ein Teil der Kohlenstoffquelle zu Beginn vorgelegt und die Kohlenstoffquelle wird über den Verlauf der Fermentation zugefüttert. Besonders bevorzugt ist dabei eine Ausführungsform, bei der ein Teil der Kohlenstoffquelle vorgelegt wird und ein Teil gefüttert wird. Besonders bevorzugt wird die Kohlenstoffquelle Glukose in einer Konzentration von 10 - 30 g/l vorgelegt, die Fütterung gestartet, wenn die Konzentration im Verlauf der Fermentation auf kleiner 5 g/l abgefallen ist und so gestaltet, dass die Konzentration unter 5 g/l gehalten wird.

Wird die Expression des rekombinanten Proteins und/oder des mutierten Pal-Proteins durch einen induzierbaren Promotor kontrolliert, so erfolgt die Induktion der Expression durch Zugabe des zum Promotor passenden Induktors zum Fermentationsansatz. Als Induktor eignen sich beispielsweise Arabinose, Laktose, IPTG, Tetracyclin oder Cumate. Der Induktor kann zu einem beliebigen Zeitpunkt während der Fermentation als Einmal- oder Mehrfach-Gabe zudosiert werden. Alternativ kann der Induktor kontinuierlich zugegeben werden. Bevorzugt wird die Expression des rekombinanten Proteins durch Zugabe von IPTG und die Expression des mutierten Pal-Proteins durch Zugabe von Arabinose induziert. Besonders bevorzugt wird IPTG als Einmalgabe zudosiert und die Induktion der Expression des mutierten Pal-Proteins so gestaltet, dass zu einem Zeitpunkt nach Abfall der Glukosekonzentration unter 5 g/l ein Gemisch aus Glukose und Arabinose zugefüttert wird. Der Anteil an Arabinose an dem Gemisch beträgt dabei bevorzugt zwischen 33 Gew.-% und 66 Gew.-%, besonders bevorzugt beträgt er 33 Gew.-%.

Die Induktion der Expression des mutierten Pal-Proteins erfolgt bevorzugt in der Phase der Kultivierung, in der nur noch wenige bis keine Zellteilungen mehr erfolgen, d.h. kurz vor bzw. am Anfang der stationären Phase der Wachstumskurve. Dieser Zeitpunkt wird dadurch bestimmt, dass die Zelldichte bestimmt als OD₆₀₀- oder CDW-Wert (s.u.) nur noch wenig bis gar nicht mehr ansteigt.

Vorzugsweise wird das Medium im Fermenter vor Inokulation gerührt und mit entkeimter Druckluft begast, dabei wird der Sauerstoff-Gehalt auf 100% Sättigung kalibriert und ein Soll-Wert für die O₂-Sättigung während der Fermentation gewählt, der zwischen 10 und 70%, bevorzugt zwischen 20 und 60% und besonders bevorzugt bei 30% dieses Werts liegt. Nach Absinken der O₂-Sättigung unter den Soll-Wert startet eine Regulationskaskade, um die O₂-Sättigung wieder an den Soll-Wert heranzuführen, wobei Gaszufuhr und Rührgeschwindigkeit angepasst werden können.

Der pH-Wert der Kultur liegt vorzugsweise zwischen pH 6 und pH 8. Bevorzugt wird ein pH-Wert zwischen 6,5 und 7,5 eingestellt, besonders bevorzugt wird der pH-Wert der Kultur zwischen 6,8 und 7,2 gehalten.

Die Temperatur der Kultur beträgt vorzugsweise zwischen 15 und 45 °C. Bevorzugt ist ein Temperaturbereich zwischen 20 und 40 °C, besonders bevorzugt ist ein Temperaturbereich zwischen 25 und 35 °C, ganz besonders bevorzugt beträgt die Temperatur 30 °C.

Erfindungsgemäß wird das Fermentationsmedium nach der Fermentation von den Zellen abgetrennt und rekombinante Proteine werden aus dem Fermentationsmedium isoliert. Dies kann durch übliche Methoden geschehen, wie sie im Stand der Technik bekannt sind. Üblicherweise werden in einem ersten Schritt durch Separationsmethoden wie Zentrifugation oder Filtration die Zellen von den in das Kulturmedium freigesetzten rekombinanten Proteinen abgetrennt. Die rekombinanten Proteine können dann z.B. durch Ultrafiltration aufkonzentriert werden.

Durch die Expression des mutierten Pal-Proteins wird die Freisetzung von rekombinanten Proteinen in den Kulturüberstand verbessert. Durch die verbesserte Freisetzung lassen sich rekombinante Proteine in erhöhter Ausbeute isolieren.

Unter erhöhter Ausbeute ist zu verstehen, dass bevorzugt mindestens 110%, besonders bevorzugt mindestens 150% und insbesondere bevorzugt mindestens 200% der Menge an rekombinantem Protein in das Kulturmedium freigesetzt wird, die nach dem gegenwärtigen Stand der Technik mit einem Wildtyp-Bakterienstamm enthaltend ein Gen für das rekombinante Protein oder einem Wildtyp-Bakterienstamm enthaltend ein Gen für das rekombinante Protein und exprimierend zusätzlich ein Protein zur Destabilisierung der bakteriellen Zellhülle produziert werden kann. Das heißt, die Ausbeute an rekombinantem Protein, das in das Kulturmedium freigesetzt wird, ist bevorzugt mindestens 1,1-mal, besonders bevorzugt mindestens 1,5-mal und insbesondere bevorzugt mindestens 2-mal so hoch wie die Ausbeute, die mit entsprechenden Bakterienstämmen aus dem Stand der Technik erzielt werden kann.

So zeigt Beispiel 1, dass im Vergleich zum entsprechenden Wildtyp- oder einem TolAIII-exprimierenden Bakterienstamm im gleichen Kulturvolumen bei Expression von PalΔ₂₂₋₂₇ oder Pal22A mehr als 2-3mal so viel CGTase im Zellüberstand produziert werden kann (s. Tabelle 1). Auch in Beispiel 2 ist die Produktion an CGTase der PalΔ₂₂₋₂₇-exprimierenden Mutante gegenüber dem entsprechenden Wildtyp- oder TolAIII-exprimierenden Bakterienstamm um einen Faktor von 2-3 erhöht (s. Tabelle 2). Ebenso bestätigt Beispiel 3 (s. Tabelle 3) eine deutliche Erhöhung.

Ein weiterer Vorteil der beschriebenen Pal-Mutanten ist, dass gleichzeitig die Zelllyse bei einer Kulturdauer von 5-24 h nur gering gegenüber entsprechenden Wildtypzellen erhöht ist. Wan und Baneyx (1998, s.o.) zeigen, dass TolAIII-Überproduktion bereits nach wenigen Stunden zu einer ca. um den Faktor 1400-3000 geringeren CFU-Zahl im Vergleich zu uninduzierten Zellen führt und schließen, dass die Langzeit-Lebensfähigkeit der Bakterienzellen durch Beeinflussung der äußeren Membran deutlich sinkt. Dagegen sinkt 24 h nach Induktion der Expression des mutierten PalΔ₂₂₋₂₇-Proteins die CFU-Zahl im Vergleich zum entsprechenden Wildtyp-Bakterienstamm nur um den Faktor 2 (s. Beispiel 3).

Mit Hilfe der Coexpression des mutierten Pal-Proteins in einem Bakterienstamm, bevorzugt in *E. coli,* können auch Fab-Antikörperfragmente extrazellulär hergestellt werden. Dabei muss die Bakterienzelle die entsprechenden Fragmente der leichten Kette, welche die Domänen VL und CL umfasst, und der schweren Kette, welche die Domänen VH und CH1 umfasst, des Antikörpers gleichzeitig synthetisieren und dann in das Periplasma sekretieren. Außerhalb des Cytoplasmas erfolgt dann die Assemblierung der beiden Ketten zum funktionellen Fab-Fragment. Die entsprechenden ORFs können in verschiedenen Genen liegen. Es ist bevorzugt, dass die ORFs codierend Antikörperfragmente der leichten und schweren Kette in einem Operon organisiert sind. Durch gleichzeitige Produktion des erfindungsgemäß mutierten Pal-Proteins werden die schwere und die leichte Kette des Antikörpers in erhöhter Konzentration in das Fermentationsmedium freigesetzt.

Das Verfahren hat den großen Vorteil, dass es zur Produktion rekombinanter Proteine, die eine lange Kulturdauer verlangen, sowie für die Hochzelldichte-Fermentation geeignet ist. Dadurch lassen sich komplexe, eukaryontische Proteine als rekombinante Proteine herstellen.

Bevorzugt ist das Verfahren dadurch gekennzeichnet, dass nach dem Abtrennen des Fermentationsmediums die rekombinanten Proteine aus dem Fermentationsmedium aufgereinigt werden.

Rekombinante Proteine können über Standardmethoden wie Fällung oder Chromatographie weiter gereinigt werden. Besonders bevorzugt sind hierbei Methoden, wie Affinitätschromatographie, bei der die bereits korrekt gefaltete native Konformation des Proteins ausgenutzt wird.

Bevorzugt ist das Verfahren dadurch gekennzeichnet, dass die Expression des mutierten Pal-Proteins induziert wird. Das bedeutet, dass entweder die Expression des mutierten Pal-Proteins oder die Expression des rekombinanten Proteins und die Expression des mutierten Pal-Proteins induziert wird. Das heißt, dass in dieser bevorzugten Ausführungsform die Expression des mutierten Pal-Proteins in jedem Fall unter Kontrolle eines induzierbaren Promotors steht. Dagegen kann die Expression des rekombinanten Proteins unter Kontrolle eines konstitutiven oder eines induzierbaren Promotors stehen. Besonders bevorzugt stehen sowohl der ORF codierend das rekombinante Protein, als auch der ORF codierend das mutierte Pal-Protein unter Kontrolle eines induzierbaren Promotors, insbesondere bevorzugt von unterschiedlich induzierbaren Promotoren. Bevorzugt ist daher die Expression des mutierten Pal-Proteins und die des rekombinanten Proteins induzierbar.

Da die Promotoren der Gene codierend das rekombinante Protein und das mutierte Pal-Protein bevorzugt unabhängig voneinander induziert werden können, kann für die Expression des rekombinanten Proteins und für die Expression des mutierten Pal-Proteins unabhängig voneinander der optimale Zeitpunkt gewählt werden.

Durch die Verwendung induzierbarer Promotoren kann die Expression der entsprechenden Proteine zu einem beliebigen Zeitpunkt der Fermentation induziert werden. Bevorzugt wird die Expression des mutierten Pal-Proteins nach der Induktion der Expression des rekombinanten Proteins induziert. Besonders bevorzugt wird die Expression des mutierten Pal-Proteins mindestens 1 Stunde, insbesondere bevorzugt mindestens 2 Stunden, darüber hinaus besonders bevorzugt 15 bis 24 Stunden und im speziellen bevorzugt 19 Stunden nach der Induktion der Expression des rekombinanten Proteins induziert.

Die Induktion der Expression des rekombinanten Proteins sowie des mutierten Pal-Proteins wird durch Zugabe des Induktors zum Kulturmedium ausgelöst, wobei der Induktor entsprechend des verwendeten Gens gewählt werden muss. In der bevorzugten Ausführungsform, in der das mutierte Pal-Gen einen Arabinose (ara)-Promotor enthält, wird die Expression des mutierten Pal-Proteins durch Zugabe des Induktors Arabinose zur Kultur induziert. In der bevorzugten Ausführungsform, in der das rekombinante Gen einen tac-Promotor enthält, wird die Expression des rekombinanten Proteins durch Zugabe des Induktors Laktose oder des Laktose-Analogons IPTG zur Kultur induziert.

Erfindungsgemäß ist auch nach Induktion der Expression des mutierten Pal-Proteins und weiterer Kultur der Zellen die Zelldichte des Stammes vergleichbar zu einem Bakterienstamm, der kein mutiertes Pal-Protein exprimiert. Insbesondere kommt es nicht zu einer starken Abnahme der optischen Dichte bedingt durch Zelllyse und nur zu einer geringen Abnahme der Lebendzellzahl gemessen als Kolonie-bildende Einheiten (CFUs).

Die gemessene optische Dichte (OD) ist umso höher, je höher die Zelldichte ist. Bakterienstämme mit destabilisierter Zellhülle zeigen im allg. höhere Zelllyse. Bei höherer Zelllyse sinkt die Zelldichte und folglich auch der OD-Wert.

Die Erfindung hat den Vorteil, dass rekombinante Proteine in das Kulturmedium freigesetzt werden, ohne dass es zu starker Lyse der Zellen des Bakterienstamms kommt. Das erfindungsgemäße Verfahren zeichnet sich daher dadurch aus, dass es sowohl zur Produktion komplexer, eukaryontischer Proteine mit entsprechend langer Kulturdauer, als auch für die Hochzelldichte-Fermentation geeignet ist.

Im Rahmen dieser Erfindung werden Fermentationen, in denen Zelltrockengewichte >50 g/l erreicht werden, als Hochzelldichte-Fermentation betrachtet. Dies reflektiert sich auch in der Literatur (Bruschi et al. 2014, Microb. Cell Fact. 13:99; Shokri und Larsson 2004, Microb. Cell Fact. 3:9; Knabben et al. 2010, J. Biotechnol. 150, S. 73-79).

Eine lange Kulturdauer bedeutet, dass die Kultivierungszeit mindestens 24 h, bevorzugt mindestens 48 h und besonders bevorzugt mindestens 72 h beträgt.

Eine geringere (bzw. nur gering erhöhte oder keine starke) Zelllyse bedeutet im Rahmen der Erfindung, dass der OD₆₀₀-, CDW-Wert oder der Wert für die Lebendzellzahl der Kultur des Bakterienstammes, die bei einer Kulturdauer von vorzugsweise 5-24 h nach Induktion der Expression des mutierten Pal-Proteins im Vergleich zum Wildtyp-Bakterienstamm, der diese Pal-Mutation nicht trägt, bestimmt wird, nur wenig bis gar nicht verringert ist. Insbesondere zur Kultur von Bakterienstämmen enthaltend Modifikationen zur Destabilisierung ihrer Zellhülle, deren Zelllyse wie von Wan und Baneyx (1998, s.o.) für Zellen exprimierend das TolAIII-Protein gezeigt, deutlich erhöht ist, haben die Bakterienstämme exprimierend das mutierte Pal-Protein einen großen Vorteil.

Bevorzugt ist daher eine Zellkultur aus dem erfindungsgemäßen Fermentationsverfahren dadurch gekennzeichnet, dass sie 5 bis 24 Stunden, besonders bevorzugt 7 Stunden nach Induktion der Expression des mutierten Pal-Proteins eine optische Dichte bestimmt bei 600 nm (OD₆₀₀) aufweist, die um höchstens 20%, besonders bevorzugt höchstens 10% und insbesondere bevorzugt höchstens 5% geringer ist, als der OD₆₀₀-Wert einer Zellkultur zum gleichen Zeitpunkt aus einem Fermentationsverfahren welches sich vom erfindungsgemäßen Verfahren nur dadurch unterscheidet, dass ein Bakterienstamm kultiviert wird, der sich vom erfindungsgemäßen Bakterienstamm nur dadurch unterscheidet, dass er den ORF codierend das mutierte Pal Protein nicht enthält. Die optische Dichte der Zellkultur bei 600 nm wird spektrophotometrisch bestimmt.

Die optische Dichte der Zellkultur bestimmt mit Hilfe eines Spektrophotometers bei 600 nm ist abhängig von der Menge an Zellen je Volumeneinheit (Zellkonzentration, Zelldichte), die wiederum ein Maß für die Zellteilungsaktivität sowie ein Maß für die Zelllyse ist, wobei Zelllyse zu einer Abnahme der Zelldichte führt.

Alternativ kann die Zellmasse auch als Zelltrockenmasse (CDW) bestimmt werden, indem eine definierte Kulturmenge durch Filtrieren oder Zentrifugieren, bevorzugt Zentrifugieren, isoliert, anschließend getrocknet und gewogen wird.

Eine weitere Alternative, die einen Vergleich der Vitalität des Bakterienstamms, der das mutierte Pal-Protein exprimiert, mit dem Bakterienstamm, der kein mutiertes Pal-Protein exprimiert, zulässt, ist die Bestimmung der Lebendzellzahl als "colony forming units" (CFU) bezogen auf ein definiertes Kulturvolumen. Das experimentelle Vorgehen ist in Wan und Baneyx (1998, s.o.) beschrieben. Die Einheit CFU gibt die vermehrungsfähigen Zellen in der Kultur an. Die Lebendzellzahl bei einem Bakterienstamm, exprimierend das mutierte Pal-Protein und ein rekombinantes Protein, ist höchstens um Faktor 100, bevorzugt höchstens um Faktor 10, besonders bevorzugt höchstens um Faktor zwei gegenüber einem Bakterienstamm, der kein mutiertes Pal-Protein exprimiert, verringert.

Dagegen beschreiben Wan und Baneyx (1998, s.o.) in Tabelle 2, dass 3 Stunden nach Induktion der TolAIII-Expression mit IPTG die Lebendzellzahl bestimmt als Anzahl CFU/ml im Wildtyp-Bakterienstamm weiter steigt, während sie im TolAIII-exprimierenden Bakterienstamm deutlich reduziert ist. Die Autoren schließen, dass TolAIII-Überexpression zwar in der logarithmischen Wachstumsphase des Bakterienstamms einen geringeren Einfluss auf das Bakterienwachstum hat, aber auf längere Zeit betrachtet durch Permeabilisierung der äußeren Zellmembran die Lebensfähigkeit der Bakterienzellen deutlich senkt.

Die nur geringfügige Beeinflussung der Zellintegrität durch die Expression des mutierten Pal-Proteins ist ein weiterer wesentlicher Vorteil der Erfindung. Durch die bevorzugte Klonierung des ORFs codierend das mutierte Pal-Protein unter einen induzierbaren Promotor kann zudem die Expression des mutierten Pal-Proteins kontrolliert werden und so die Kulturzeit, in der der gewünschte Effekt auftritt, eng begrenzt werden, was wiederum mögliche Auswirkungen auf die Vitalität der Bakterienzellen begrenzt.
Fig. 1 zeigt die Plasmidkarte des Plasmids pCGT-Pal.
Fig. 2 zeigt die Plasmidkarte des Plasmids pCGT-TolAIII.
Fig. 3 zeigt die Plasmidkarte des Plasmids pJF118ut-CD154.
Fig. 4 zeigt die Plasmidkarte des Plasmids pJF118ut-CD154-Pal.
Fig. 5 zeigt die Plasmidkarte des Plasmids pCGT-Pal22A.

Die in den Figuren verwendeten Abkürzungen stehen für DNS-Regionen, die die folgenden Funktionen codieren:
- tac p/o:: tac-Promotor/Operator
- pBAD p/o:: Arabinose-Promotor/Operator
- bla:: β-Lactamase-Gen (Ampicillin-Resistenz)
- TcR:: Tetracyclin-Resistenz
- lacIq:: Repressor des tac-Promotors
- cgt-SP:: Signalpeptid der CGTase
- CGTase:: Cyclodextrin-Glycosyl-Transferase
- ColE1:: Replikationsursprung
- phoA-SP:: phoA-Signalpeptid
- AFA-SP:: Derivat des Signalpeptids der CGTase
- rrnB-Terminator:: Terminatorregion des rrnB-Gens
- trpA-Terminator:: Terminatorregion des trpA-Gens
- Pal:: Peptidoglykan-assoziiertes Lipoprotein
- Pal-SP:: Signalpeptid von Pal
- TolAIII:: Domäne des TolA-Proteins
- OmpA-SP:: Signalpeptid von OmpA
- HisTag:: Histidin-Tag
- light chain:: Antikörperfragment umfassend die Domänen VL und CL
- heavy chain:: Antikörperfragment umfassend die Domänen VH und CH1
- ScaI/MauBI/EcoRI:: Schnittstellen der entsprechenden Restriktionsendonukleasen

### Beispiele

Die Erfindung wird im Folgenden anhand von Ausführungsbeispielen näher beschrieben, ohne dadurch beschränkt zu werden.

Sämtliche eingesetzten molekularbiologischen Verfahren, wie Polymerase-Ketten-Reaktion (PCR), Gensynthese, Isolierung und Reinigung von DNS, Modifikation von DNS durch Restriktionsenzyme und Ligase, Transformation usw. wurden in der dem Fachmann bekannten, in der Literatur beschriebenen oder von den jeweiligen Herstellern empfohlenen Art und Weise durchgeführt.

### Beschreibung der Plasmide:

### pCGT:

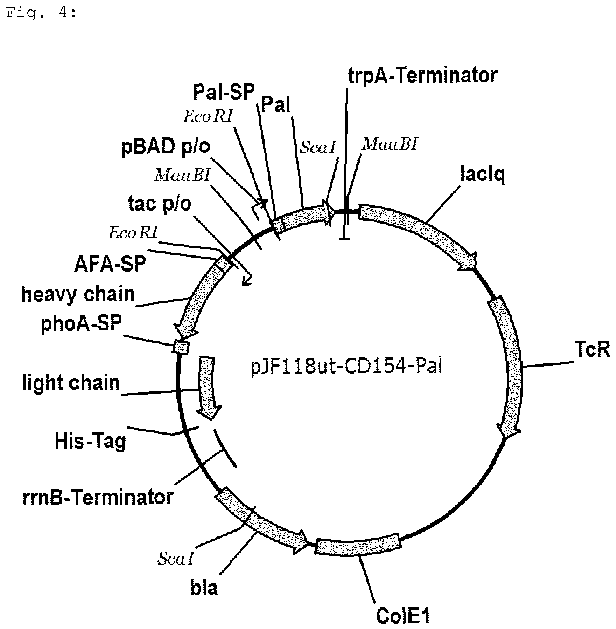

Die Herstellung des Plasmids pCGT ist in Beispiel 4 von US 2008/0254511 A1 beschrieben, die Plasmidkarte in Fig. 4 von US 2008/0254511 A1 angegeben.

Im Wesentlichen enthält das Plasmid neben dem Gen für die Resistenz gegen Tetracyclin u.a. auch das Strukturgen der Cyclodextrin-Glycosyl-Transferase (CGTase) aus *Klebsiella pneumoniae* M5a1 einschließlich der nativen CGTase-Signalsequenz. Die Expression des CGTase-Gens steht unter der Kontrolle des tac-Promotors.

### pCGT-Pal:

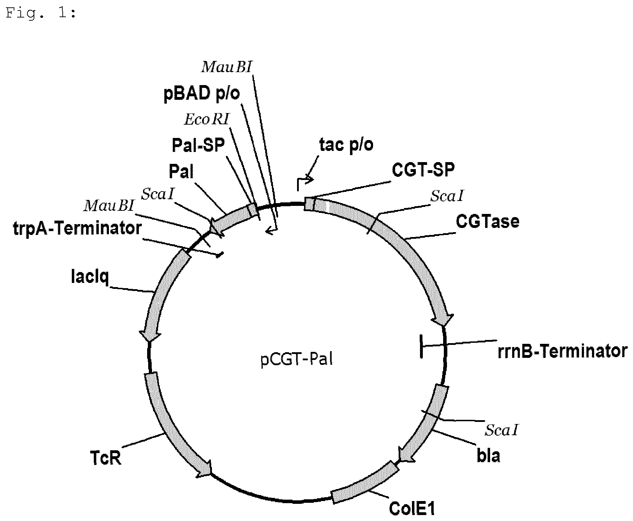

Um pCGT-Pal (Plasmidkarte s. Fig. 1) zu erhalten, wurde ein DNS-Fragment mittels Gensynthese von der Firma eurofins Genomics hergestellt. Dieses DNS-Fragment xI (angegeben in SEQ ID Nr. 1) enthielt:
- die Nukleotide 1136-1304 aus GenBank-Eintrag X81837.1, enthaltend den Arabinose-Promotor (pBAD-Promotor), sowie die Operatoren O1 und I2+I1 und die CAP-Bindestelle (Nukleotide 10-178 von SEQ ID Nr. 1),
- die Shine-Dalgarno-Sequenz (Nukleotide 203-208 von SEQ ID Nr. 1) und
- ein Nukleotid-Fragment, codierend für eine Fusion aus
   i der Signalsequenz des Peptidoglykan-assoziierten Lipoproteins aus *E. coli* K12 (Nukleotide 136-198 aus GenBank-Eintrag X05123.1 codierend für die Aminosäuren -21 bis - 1, Nukleotide 216-278 von SEQ ID Nr. 1),
   ii den Nukleotiden 217-654 aus GenBank-Eintrag X05123.1 codierend die Aminosäuren 7-152 des Peptidoglykan-assoziierten Lipoproteins aus *E. coli* K12 (Nukleotide 279-716 von SEQ ID Nr. 1) und
   iii dem Terminator des trpA-Gens aus *E*. *coli* (Nukleotide 749-774 von SEQ ID Nr. 1).

Dieses DNS-Fragment xI wurde mit dem Restriktionsenzym MauBI geschnitten und mit dem Expressionsvektor pCGT, der mit demselben Restriktionsenzym geschnitten worden war, ligiert. Die Klonierung erfolgte ungerichtet, es wurde jedoch bevorzugt mit dem Plasmid gearbeitet, in dem das DNS-Fragment in der entgegengesetzten Leserichtung wie das Gen codierend für die CGTase eingefügt war, wobei der Nachweis über das Restriktionsmuster des Restriktionsenzyms ScaI und Sequenzieren erfolgte. Dieses Plasmid wurde als pCGT-Pal bezeichnet und codiert das Protein PalΔ₂₂₋₂₇, das synonym auch mit PalD22-27 bezeichnet wird (angegeben in SEQ ID Nr. 5).

### pCGT-Pal22A:

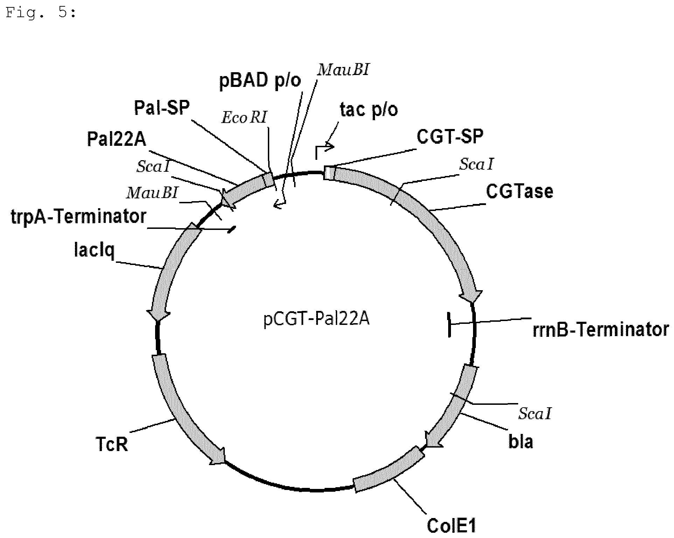

Um pCGT-Pal22A (Plasmidkarte s. Fig. 5) zu erhalten, wurde ein DNS-Fragment mittels Gensynthese von der Firma eurofins Genomics hergestellt. Dieses DNS-Fragment xIA (angegeben in SEQ ID Nr. 6) enthielt:
- die Nukleotide 1136-1304 aus GenBank-Eintrag X81837.1, enthaltend den Arabinose-Promotor (pBAD-Promotor), sowie die Operatoren O1 und I2+I1 und die CAP-Bindestelle (Nukleotide 10-178 von SEQ ID Nr. 6),
- die Shine-Dalgarno-Sequenz (Nukleotide 203-208 von SEQ ID Nr. 6) und
- ein Nukleotid-Fragment, codierend für eine Fusion aus
   i der Signalsequenz des Peptidoglykan-assoziierten Lipoproteins aus *E. coli* K12 (Nukleotide 136-198 aus GenBank-Eintrag X05123.1 codierend für die Aminosäuren -21 bis - 1, Nukleotide 216-278 von SEQ ID Nr. 6),
   ii den Aminosäuren 1-152 des Peptidoglykan-assoziierten Lipoproteins aus *E. coli* K12 (vgl. GenBank: X05123.1, Nukleotide 279-734 von SEQ ID Nr. 6), wobei die Aminosäure Cystein an Position 1 gegen ein Alanin ausgetauscht wurde und
   iii dem Terminator des trpA-Gens aus *E. coli* (Nukleotide 767-792 von SEQ ID Nr. 6).

Dieses DNS-Fragment xIA wurde mit dem Restriktionsenzym MauBI geschnitten und mit dem Expressionsvektor pCGT, der mit demselben Restriktionsenzym geschnitten worden war, ligiert. Die Klonierung erfolgte ungerichtet, es wurde jedoch bevorzugt mit dem Plasmid gearbeitet, in dem das DNS-Fragment in der entgegengesetzten Leserichtung wie das Gen codierend für die CGTase eingefügt war, wobei der Nachweis über das Restriktionsmuster des Restriktionsenzyms ScaI und Sequenzieren erfolgte. Dieses Plasmid wurde als pCGT-Pal22A bezeichnet und codiert das Protein Pal22A (angegeben in SEQ ID Nr. 7).

### pCGT-TolAIII:

Die Überexpression von TolAIII entspricht dem Stand der Technik (Wan und Baneyx 1998, s.o.), daher wurde pCGT-TolAIII als Vergleichsbeispiel stellvertretend für den Stand der Technik gewählt, in dem die äußere Zellhülle eines Bakteriums, enthaltend dieses Plasmid und exprimierend TolAIII, destabilisiert und dadurch die Freisetzung von rekombinanten Proteinen erhöht wird.

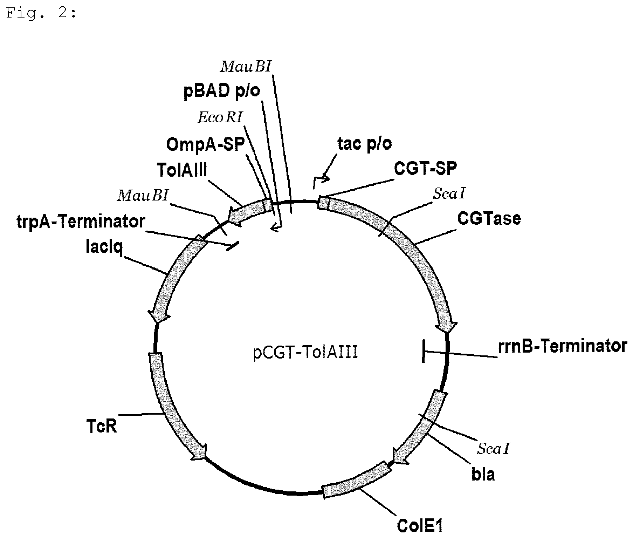

Um pCGT-TolAIII (Plasmidkarte s. Fig. 2) zu erhalten, wurde ein DNS-Fragment per Gensynthese von der Firma eurofins Genomics hergestellt. Dieses DNS-Fragment xII (angegeben in SEQ ID Nr. 2) enthielt:
- die Nukleotide 1136-1304 aus GenBank-Eintrag X81837.1, enthaltend den Arabinose-Promotor (pBAD-Promotor), sowie die Operatoren O1 und I2+I1 und die CAP-Bindestelle (Nukleotide 10-178 von SEQ ID Nr. 2),
- die Shine-Dalgarno-Sequenz (Nukleotide 203-208 von SEQ ID Nr. 2) und
- ein Nukleotidfragment, codierend für eine Fusion aus
   i der Signalsequenz von ompA aus *E. coli* K12 (Nukleotide 216-278 von SEQ ID Nr. 2),
   ii den Aminosäuren 291-421 des TolA-Proteins aus *E. coli* K12 (codierend für TolAIII, Nukleotide 279-671 von SEQ ID Nr. 2) und
   iii dem Terminator des trpA-Gens aus *E. coli* (Nukleotide 714-729 von SEQ ID Nr. 2).

Dieses DNS-Fragment xII wurde mit dem Restriktionsenzym MauBI geschnitten und mit dem Expressionsvektor pCGT (s.o.), der mit demselben Restriktionsenzym geschnitten worden war, ligiert. Die Klonierung erfolgte ungerichtet, es wurde jedoch bevorzugt mit dem Plasmid gearbeitet, in dem das DNS-Fragment in der entgegengesetzten Leserichtung wie das Gen codierend für die CGTase eingefügt war, wobei der Nachweis über das Restriktionsmuster der Restriktionsenzyme ScaI und EcoRI oder Sequenzieren erfolgte. Dieses Plasmid wurde als pCGT-TolAIII bezeichnet.

### pJF118ut-CD154:

Als Ausgangsvektor für die Klonierung und Expression der Gene des Fab-Fragments des humanisierten monoklonalen anti-CD154-Antikörpers 5c8, dessen Sequenz in Karpusas et al. 2001 (Structure 9, S. 321-329) veröffentlicht ist, diente das in US 2008/076157 A beschriebene Plasmid pJF118ut. pJF118ut ist ein Derivat des bekannten Expressionsvektors pKK223-3 (Amersham Pharmacia Biotech) und bei der DSMZ-Deutsche Sammlung von Mikroorganismen und Zellkulturen GmbH (Braunschweig) unter der Nummer DSM 18596 hinterlegt.

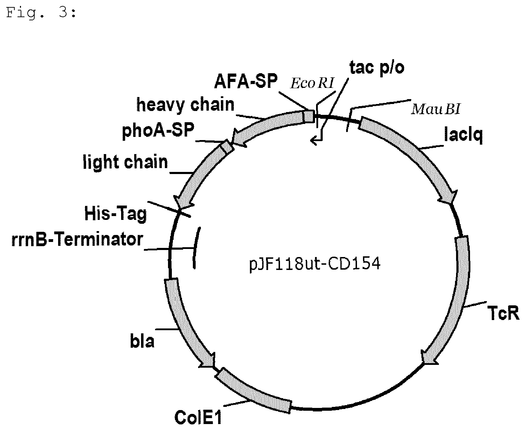

Um pJF118ut-CD154 (Plasmidkarte s. Fig. 3) zu erhalten, wurde ein DNS-Fragment per Gensynthese von der Firma eurofins Genomics hergestellt. Dieses DNS-Fragment xIII (angegeben in SEQ ID Nr. 3) umfasste eine Fusion bestehend aus:
i der in US 2008/076157 unter der SEQ ID Nr. 2 offenbarten Signalsequenz, abgeleitet aus der Signalsequenz einer CGTase aus *Klebsiella pneumoniae* M5a1 (Nukleotide 25-114 von SEQ ID Nr. 3) und
ii dem Leserahmen für die schwere Kette (V_{H}-C_{H}1-Domänen) des Fab-Fragments des humanisierten monoklonalen anti-CD154-Antikörpers 5c8, codierend die Aminosäuren 1-221 der in Karpusias et al. 2001 in Fig. 3 veröffentlichten Sequenz (Nukleotide 115-777 von SEQ ID Nr. 3),
iii der phoA-Signalsequenz (Nukleotide 800-862 von SEQ ID Nr. 3),
iv dem Leserahmen für die leichte Kette (V_{L}-C_{L}-Domänen) des Fab-Fragments des humanisierten monoklonalen anti-CD154-Antikörpers 5c8, wie von Karpusias et al. 2001 in Fig. 3 veröffentlicht (Nukleotide 863-1516 von SEQ ID Nr. 3) und
v den Nukleotiden 1517-1546 von SEQ ID Nr. 3, codierend einen 4-Aminosäure-langen Linker und einen Hexa-Histidin-Tag.

Dieses DNS-Fragment xIII wurde mit den Restriktionsenzymen EcoRI und PdmI geschnitten und mit dem Expressionsvektor pJF118ut, der mit EcoRI und SmaI geschnitten worden war, ligiert. Das resultierende Plasmid, bei dem die Expression der Gene für die schwere und leichte Kette des Fab-Fragments unter der Kontrolle des tac-Promotors standen, wurde mit pJF118ut-CD154 bezeichnet.

### pJF118ut-CD154-Pal:

In das Plasmid pJF118ut-CD154 wurde das DNS-Fragment xI codierend für die Pal-Variante unter Kontrolle des Arabinose-Promotors und flankiert vom trpA-Terminator, wie für pCGT-Pal oben beschrieben, über die MauBI Schnittstelle eingeführt. Die Klonierung erfolgte ungerichtet, es wurde jedoch bevorzugt mit dem Plasmid gearbeitet, in dem das DNS-Fragment xI in der entgegengesetzten Leserichtung wie das DNS-Fragment xIII codierend für CD154 eingefügt war, wobei der Nachweis über das Restriktionsmuster des Restriktionsenzyms ScaI und Sequenzieren erfolgte. Dieses Plasmid wurde als pJF118ut-CD154-Pal bezeichnet (Plasmidkarte s. Fig. 4).

### Beispiel 1: Produktion der Cyclodextrin-Glycosyl-Transferase (CGTase) im Schüttelkolben

Für die Produktion der CGTase aus *Klebsiella pneumoniae* M5a1 wurde der *E*. *coli-Stamm* W3110 (ATCC 27325) mit den Plasmiden pCGT, pCGT-Pal, pCGT-Pal22A oder pCGT-TolAIII nach gängigen Methoden (z.B. TSS-Transformation) transformiert. Die Selektion auf plasmidhaltige Zellen erfolgte mittels Tetracyclin (20 mg/l). Die *E*. *coli-*Stämme wurden mit W3110/pCGT, W3110/pCGT-Pal, W3110/pCGT-Pal22A und W3110/pCGT-TolAIII bezeichnet.

Die transformierten Stämme wurden in 10 ml LB-Medium (5 g/l Hefeextrakt (Oxoid LP0021), 10 g/l Trypton (Oxoid LP0042), 5 g/l NaCl), zusätzlich enthaltend 1 ml/l Spurenelementlösung (0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl2 x 4 H₂O; 0,3 g/l ZnSO₄ x 7 H₂O), 3 g/l Glukose, 10 g/l Laktose, 0,55 g/l CaCl₂ und 20 mg/l Tetracyclin, bei 30 °C angezogen. Bei dem verwendeten Medium handelte es sich um ein Autoinduktionsmedium, d.h. es musste kein zusätzlicher Induktor für den tac-Promotor zugegeben werden. Nach Verstoffwechselung der im Medium enthaltenen Glukose konnte die Laktose durch die Zellen aufgenommen werden. Dadurch kam es zur Induktion der Proteinexpression ausgehend vom tac-Promotor. Von allen vier Plasmiden wurde CGTase produziert. Durch Zugabe von 0,2 % (w/v) Arabinose nach 48 h zum Kulturmedium wurde von den Plasmiden pCGT-Pal und pCGT-Pal22A zusätzlich die Expression der ebenfalls auf dem Plasmid enthaltenen und unter Kontrolle des Arabinose-Promotors stehenden Pal-Varianten induziert, vom Plasmid pCGT-TolAIII dagegen zusätzlich die Expression von TolAIII. Auch die das Plasmid pCGT enthaltenden Kontrollkulturen wurden zur besseren Vergleichbarkeit entsprechend mit Arabinose versetzt.

Nach 72 h Kulturdauer wurden Proben entnommen, die Zellen durch Zentrifugation vom Kulturmedium abgetrennt und der CGTase-Gehalt im Kulturüberstand anhand der Menge an enzymatisch aus Stärke produziertem Cyclodextrin (CD) durch folgenden Enzymtest bestimmt:

| | |
|---|---|
| Testpuffer: | 5 mM Tris-HCl-Puffer, 5 mM CaCl₂ x 2 H₂0, pH 6,5 |
| Substratlösung: | 10 %ige Stärkelösung (Merck Nr.1.01252) in Testpuffer, pH 6,5 |
| Testansatz: | 0,2 ml Substratlösung + 0,2 ml abzentrifugierter (5 min, 12 000 rpm) Kulturüberstand |
| Reaktionstemperatur: | 40°C |

### Enzymtest:

* Vortemperieren von Substratlösung und abzentrifugiertem Kulturüberstand (ca. 5 min bei 40°C)
* Herstellen des Testansatzes durch rasches Mischen (Whirl-Mixer) von Substratlösung und abzentrifugiertem Kulturüberstand, wobei der abzentrifugierte Kulturüberstand gegebenenfalls mit Testpuffer verdünnt wird, so dass in der anschließenden HPLC-Analytik ein Wert von 0,9-1,5 g/l CD bestimmt wird;
* Inkubation für 3 min bei 40°C
* Stoppen der Enzymreaktion durch Zugabe von 0,6 ml Methanol, rasches Mischen (Whirl-Mixer)
* Abkühlen des Ansatzes auf Eis (ca. 5 min)
* Abzentrifugieren (5 min, 12 000 rpm) und Abpipettieren des klaren Überstandes
* Analyse der Menge an produziertem CD mittels HPLC: Die Analyse wurde an einem Agilent HP 1100 HPLC-System mit einer Nucleodur 100-3 NH2-RP Säule (150 mm x 4,6 mm, Macherey-Nagel) und 64% Acetonitril in Wasser(v/v) als Fließmittel, bei einer Flussrate von 2,1 ml/min durchgeführt. Die Detektion erfolgte über einen RI-Detektor (1260 Infinity RI, Agilent) und die Quantifizierung wurde anhand der Peakfläche und einem α-CD-Standard (Cavamax W6-8 Pharma, Wacker Chemie AG) vorgenommen.

Berechnung der Enzymaktivität: A = G*V1*V2/(t*MG) [U/ml]
A = Aktivität,
G = Gehalt an CD in mg/l
V1 = Verdünnungsfaktor im Testansatz
V2 = Verdünnungsfaktor des Kulturüberstands vor dem Einsatz im Test;
   wenn unverdünnt gilt: V2 = 1
t = Reaktionszeit in min
MG = Molekulargewicht in g/mol (MG_{CD} = 973 g/mol)
1 Unit (U) ≙ 1 µmol/l Produkt (CD) / min

Tabelle 1 zeigt die jeweils erzielten CGTase-Ausbeuten.

**Tabelle 1: CGTase-Ausbeuten im Schüttelkolben-Überstand nach 72 h Kultivierung.**

| Stamm | Schüttelkolben: CGTase (U/ml) |
|---|---|
| W3110/pCGT | 39 |
| W3110/pCGT-Pal | 134 |
| W3110/pCGT-Pal22A | 95 |
| W3110/pCGT-TolAIII | 42 |

### Beispiel 2: Fermentative Produktion der Cyclodextrin-Glycosyl-Transferase (CGTase) im Rührkesselfermenter

Für die Produktion der Cyclodextrin-Glycosyl-Transferase (CGTase) aus Klebsiella pneumoniae M5a1 wurden die in Beispiel 1 beschriebenen Stämme W3110/pCGT, W3110/pCGT-Pal und W3110/pCGT-TolAIII verwendet.

Die CGTase-Produktion erfolgte in Rührkesselfermentern.

Der mit 1,2 1 des Fermentationsmediums (1,5 g/l KH₂PO₄; 5 g/l (NH₄)₂SO₄; 0,5 g/l MgSO₄ x 7 H₂O; 0,225 g/l CaCl₂ x 2 H₂O, 0,075 g/l FeSO₄ x 7 H₂O; 1 g/l Na₃Citrat x 2 H₂O; 0,5 g/l NaCl; 1 ml/l Spurenelementlösung (0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O); 5 mg/l Vitamin B1; 3 g/l Phyton-Pepton (BD 211906); 1,5 g/l Hefeextrakt (Oxoid LP0021); 10 g/l Glukose; 20 mg/l Tetracyclin) befüllte Fermenter wurde ad 0,1 OD₆₀₀ mit einer Vorkultur, die für 7 h im Schüttelkolben im unter Beispiel 1 genannten LB-Medium kultiviert worden war, angeimpft. Damit wurde die Fermentation gestartet (Zeitpunkt 0, Fermentationsbeginn). Während der Fermentation wurde eine Temperatur von 30 °C eingestellt und der pH-Wert durch Zudosierung von NH₄OH bzw. H₃PO₄ bei einem Wert von 7,0 konstant gehalten. Glukose wurde über die Fermentation hinweg zudosiert, wobei eine Glukose-Konzentration von < 5 g/l angestrebt wurde. Die Induktion der Expression der CGTase erfolgte durch Zugabe von Isopropyl-β-D-thiogalactopyranosid (IPTG) ad 0,15 mM nach 22 h (am Ende der logarithmischen Wachstumsphase). Die Induktion der Expression der Pal-Variante erfolgte 24 h bzw. 41 h nach Fermentationsbeginn und die Induktion der Expression von TolAIII erfolgte 41 h nach Fermentationsbeginn durch Umstellung der reinen Zufütterung von Glukose auf eine konstante Glukose-Arabinose-Misch-Fütterung von 3 g/l*h im Verhältnis 2:1 Glukose:Arabinose.

Das Medium im Fermenter wurde vor Inokulation mit 400 rpm gerührt und mit 1,67 vvm (Volumen Luft pro Volumen Kulturmedium pro Minute) einer über einen Sterilfilter entkeimten Druckluft begast. Unter diesen Startbedingungen wurde der optische Sauerstoff-Sensor (VisiFerm DO225, Hamilton) vor der Inokulation auf 100% Sättigung kalibriert. Der Soll-Wert für die O₂-Sättigung während der Fermentation wurde auf 30 % dieses Werts eingestellt. Die O₂-Sättigung wurde während der Fermentation über den Sauerstoffsensor gemessen und von der Fermenter-Steuerung DCU (Digital control unit, Sartorius Stedim) erfasst. Nach Absinken der O₂-Sättigung unter den Soll-Wert wurde Software-gesteuert die Rührgeschwindigkeit kontinuierlich auf max. 1.500 rpm gesteigert, um die O₂-Sättigung wieder an den Soll-Wert heranzuführen.

Nach 48 h Fermentation wurden Proben entnommen, die Zellen durch Zentrifugation vom Fermentationsmedium abgetrennt und der CGTase-Gehalt im Fermentationsüberstand durch den Aktivitätstest, wie in Beispiel 1 beschrieben, bestimmt. Tabelle 2 zeigt die jeweils erzielten Cyclodextrin-Glycosyltransferase-Ausbeuten.

**Tabelle 2: CGTase-Ausbeuten im Fermentationsüberstand nach 48 h Kultivierung.**

| Stamm | CGTase (relative Ausbeute in %) |
|---|---|
| W3110/pCGT | 100 |
| W3110/pCGT-Pal, Induktion nach 24 h | 430 |
| W3110/pCGT-Pal, Induktion nach 41 h | 690 |
| W3110/pCGT-TolAIII | 260 |

Um den Einfluss der Expression der Pal-Variante auf das Zellwachstum zu untersuchen, wurde im Fermentationsverlauf für W3110/pCGT und W3110/pCGT-Pal (Induktion nach 41 h) das Wachstum der Zellen über die Messung der optischen Dichte bei 600 nm (OD₆₀₀) an einem Spektrophotometer (Beckman Coulter DU 730) bestimmt.

Zusätzlich wurde die Zelltrockenmasse der Kulturen bestimmt. Zur Bestimmung der Zelltrockenmasse wurden Proben, enthaltend 1 ml Fermenterkultur, in Reaktionsgefäße transferiert, deren Leergewicht zuvor bestimmt worden war. Nach Zentrifugation (5 min, 12.000 rpm) wurden die Überstände entfernt und die Zellpellets in einem Inkubator getrocknet (≥ 48 h bei 60°C). Anschließend wurden die Gefäße mit dem getrockneten Zellpellet ausgewogen und die Zelltrockenmasse (cell dry weight, CDW) aus der Differenz der Masse der Gefäße mit getrocknetem Zellpellet und der Masse der leeren Gefäße errechnet. Die Ergebnisse sind in Tabelle 3 zusammengefasst.

**Tabelle 3: Optische Dichte (OD₆₀₀) und Zelltrockenmassen (CDW) der CGTase-produzierenden Stämme im Fermenter.**

| Stamm | OD₆₀₀ | | CDW (g/l) | |
|---|---|---|---|---|
| | Kulturdauer | | Kulturdauer | |
| | 41 h | 48 h | 41 h | 48 h |
| W3110/pCGT | 142 | 153 | 51 | 55 |
| W3110/pCGT-Pal, Induktion nach 41 h | 146 | 150 | 52 | 52 |

### Beispiel 3: Produktion eines Fab-Antikörperfragments im Schüttelkolben

Die Produktion des CD154-Fab-Fragments im Schüttelkolben erfolgte analog dem in Beispiel 1 beschriebenen Verfahren mit den E. coli-Stämmen W3110/pJF118ut-CD154 und W3110/pJF118ut-CD154-Pal. Nach 72 h wurden Proben entnommen, die Zellen durch Zentrifugation vom Kulturmedium abgetrennt und der Überstand zur Bestimmung des in das Kulturmedium freigesetzten CD154-Fab-Fragments analysiert. Das Zellpellet wurde in PBS-Puffer aufgenommen und die Zellen an einem FastPrep-Homogenisator (MP Biomedicals) aufgeschlossen. Aus dem so gewonnenen Zelllysat wurde das intrazellulär vorliegende CD154-Fab-Fragment bestimmt.

Die Quantifizierung des CD154-Fab-Fragments erfolgte über einen dem Fachmann bekannten Sandwich-ELISA-Test. Dabei diente ein immobilisierter Anti-human-IgG (Fd)-Antikörper (The Binding Site, Product No. PC075) als Fänger und ein Peroxidase-konjugierter goat Anti-Human Kappa Light Chains Antikörper (Sigma, Product No. A 7164) als Detektions-Antikörper. Die Quantifizierung erfolgte durch Umsetzung des chromogenen Substrats Dako TMB+ (Dako # S1599) durch die Peroxidase und die damit einhergehende Absorptionsänderung bei 450 nm. Zur Kalibrierung des ELISA wurde das Fab-Fragment "Human Fab/Kappa" (Bethyl Laboratories, Produktnummer: P80-115) verwendet.

**Tabelle 3: Ausbeuten an CD154-Antikörper-Fragment im Kulturüberstand.**

| Stamm | CD154-Antikörper Fragment (mg/l) im Überstand |
|---|---|
| W3110/pJF118ut-CD154 | 59 |
| W3110/pJF118ut-CD154-Pal | 87 |

Neben der Produktausbeute wurde nach 72 h auch die Lebendzellzahl der Kulturen bestimmt. Hierzu wurden Proben der Kulturen 10⁵-fach mit LB-Medium in einem Endvolumen von 1 ml verdünnt und jeweils 100 µl der Verdünnung auf LB-Agarplatten enthaltend 20 mg/l Tetracyclin ausplattiert. Die gewachsenen Kolonien wurden ausgezählt und unter Berücksichtigung des Verdünnungsfaktors die Lebendzellzahl der Ausgangskulturen berechnet. Diese betrug 5,5·10⁸ Zellen/ml für den Stamm W3110/pJF118ut-CD154 und 2,7·10⁸ Zellen/ml für den Stamm W3110/pJF118ut-CD154-Pal

## Patentansprüche

1. Verfahren zur fermentativen Produktion von rekombinanten Proteinen **dadurch gekennzeichnet, dass** ein Bakterienstamm der Art *Escherichia coli* enthaltend einen offenen Leserahmen codierend für ein rekombinantes Protein unter Kontrolle eines funktionellen Promotors und einen offenen Leserahmen codierend für ein mutiertes Peptidoglykan-assoziiertes Lipoprotein (Pal-Protein) unter Kontrolle eines funktionellen Promotors in einem Fermentationsmedium kultiviert, das Fermentationsmedium nach der Fermentation von den Zellen abgetrennt und rekombinante Proteine aus dem Fermentationsmedium isoliert werden,
wobei das mutierte Pal-Protein derart mutiert ist, dass es keinen Membrananker für die äußere Zellmembran des Bakteriums besitzt und
wobei der Bakterienstamm zusätzlich das Wildtyp-Pal-Gen enthält.

2. Verfahren gemäß einem Anspruch 1, **dadurch gekennzeichnet, dass** der offene Leserahmen codierend das mutierte Pal-Protein derart mutiert ist, dass er für ein mutiertes Pal-Protein codiert, das an einer oder mehreren der Aminosäurepositionen 1 bis 6 mutiert ist.

3. Verfahren gemäß Anspruch 2, **dadurch gekennzeichnet, dass** der offene Leserahmen codierend das mutierte Pal-Protein derart mutiert ist, dass er für ein mutiertes Pal-Protein codiert, bei dem der N-terminale Cysteinrest substituiert ist.

4. Verfahren gemäß Anspruch 2, **dadurch gekennzeichnet, dass** der offene Leserahmen codierend das mutierte Pal-Protein derart mutiert ist, dass er für ein mutiertes Pal-Protein codiert, bei dem der N-terminale Cysteinrest fehlt.

5. Verfahren gemäß Anspruch 2, **dadurch gekennzeichnet, dass** der offene Leserahmen codierend das mutierte Pal-Protein derart mutiert ist, dass er für ein mutiertes Pal-Protein codiert, bei dem die Aminosäuren 1 bis 6 fehlen.

6. Verfahren gemäß einem oder mehreren der Ansprüche 1 bis 5, **dadurch gekennzeichnet, dass** das rekombinante Protein ein heterologes Protein ist.

7. Verfahren gemäß einem oder mehreren der Ansprüche 1 bis 6, **dadurch gekennzeichnet, dass** nach dem Abtrennen des Fermentationsmediums die rekombinanten Proteine aus dem Fermentationsmedium aufgereinigt werden.

8. Verfahren gemäß einem oder mehreren der Ansprüche 1 bis 7, **dadurch gekennzeichnet, dass** die Expression des mutierten Pal-Proteins induziert wird.

9. Verfahren gemäß einem oder mehreren der Ansprüche 1 bis 8, **dadurch gekennzeichnet, dass** die Expression des mutierten Pal-Proteins nach der Induktion der Expression des rekombinanten Proteins induziert wird.

## Claims

1. Method for fermentative production of recombinant proteins, **characterized in that** a bacterial strain of the species Escherichia coli containing an open reading frame encoding a recombinant protein under the control of a functional promoter and an open reading frame encoding a mutated peptidoglycan-associated lipoprotein (Pal protein) under the control of a functional promoter is cultured in a fermentation medium, the fermentation medium is removed from the cells after the fermentation, and recombinant proteins are isolated from the fermentation medium,
the mutated Pal protein having been mutated such that it has no membrane anchor for the outer cell membrane of the bacterium and
the bacterial strain additionally containing the wild-type Pal gene.

2. Method according to Claim 1, **characterized in that** the open reading frame encoding the mutated Pal protein has been mutated such that it encodes a mutated Pal protein which has been mutated at one or more of amino acid positions 1 to 6.

3. Method according to Claim 2, **characterized in that** the open reading frame encoding the mutated Pal protein has been mutated such that it encodes a mutated Pal protein in which the N-terminal cysteine residue has been substituted.

4. Method according to Claim 2, **characterized in that** the open reading frame encoding the mutated Pal protein has been mutated such that it encodes a mutated Pal protein in which the N-terminal cysteine residue is absent.

5. Method according to Claim 2, **characterized in that** the open reading frame encoding the mutated Pal protein has been mutated such that it encodes a mutated Pal protein in which amino acids 1 to 6 are absent.

6. Method according to one or more of Claims 1 to 5, **characterized in that** the recombinant protein is a heterologous protein.

7. Method according to one or more of Claims 1 to 6, **characterized in that** the recombinant proteins are purified from the fermentation medium after the removal of the fermentation medium.

8. Method according to one or more of Claims 1 to 7, **characterized in that** that the expression of the mutated Pal protein is induced.

9. Method according to one or more of Claims 1 to 8, **characterized in that** the expression of the mutated Pal protein is induced after the induction of the expression of the recombinant protein.

## Revendications

1. Procédé de production par fermentation de protéines recombinantes, **caractérisé en ce que** l'on cultive dans un milieu de fermentation une souche bactérienne de l'espèce *Escherichia coli* contenant un cadre de lecture ouvert codant pour une protéine recombinante sous le contrôle d'un promoteur fonctionnel et un cadre de lecture ouvert codant pour une lipoprotéine mutée associée à un peptidoglycane (protéine Pal) sous le contrôle d'un promoteur fonctionnel, après la fermentation, le milieu de fermentation étant séparé des cellules et les protéines recombinantes étant isolées du milieu de fermentation,
dans lequel la protéine Pal mutée est mutée de telle sorte qu'elle ne possède pas d'ancrage membranaire pour la membrane cellulaire externe de la bactérie et
dans lequel la souche bactérienne contient en outre le gène Pal de type sauvage.

2. Procédé selon la revendication 1, **caractérisé en ce que** le cadre de lecture ouvert codant pour la protéine Pal mutée est muté de telle sorte qu'il code pour une protéine Pal mutée qui a muté sur une ou plusieurs des positions d'acides aminés 1 à 6.

3. Procédé selon la revendication 2, **caractérisé en ce que** le cadre de lecture ouvert codant pour la protéine Pal mutée est muté de telle sorte qu'il code pour une protéine Pal mutée dans laquelle le résidu cystéine N-terminal est substitué.

4. Procédé selon la revendication 2, **caractérisé en ce que** le cadre de lecture ouvert codant pour la protéine Pal mutée est muté de telle sorte qu'il code pour une protéine Pal mutée dans laquelle le résidu cystéine N-terminal est absent.

5. Procédé selon la revendication 2, **caractérisé en ce que** le cadre de lecture ouvert codant pour la protéine Pal mutée est muté de telle sorte qu'il code pour une protéine Pal mutée dans laquelle les acides aminés 1 à 6 sont absents.

6. Procédé selon une ou plusieurs des revendications 1 à 5, **caractérisé en ce que** la protéine recombinante est une protéine hétérologue.

7. Procédé selon une ou plusieurs des revendications 1 à 6, **caractérisé en ce qu'**après la séparation du milieu de fermentation, les protéines recombinantes sont purifiées à partir du milieu de fermentation.

8. Procédé selon une ou plusieurs des revendications 1 à 7, **caractérisé en ce que** l'expression de la protéine Pal mutée est induite.

9. Procédé selon une ou plusieurs des revendications 1 à 8, **caractérisé en ce que** l'expression de la protéine Pal mutée est induite après l'induction de l'expression de la protéine recombinante.
